# EUROPEAN PATENT SPECIFICATION

(11) **EP 1 962 587 B1**
(45) Date of publication and mention of the grant of the patent: **17.02.2010**
(21) Application number: 07794736.4
(22) Date of filing: 10.05.2007
(51) Int. Cl.: A01N 25/06, A01P 17/00, A61K 8/04, A61Q 17/02, A01N 25/08, A01N 25/10

(54) **INSECT CONTROL COMPOSITIONS**
ZUSAMMENSETZUNGEN ZUR INSEKTENBEKÄMPFUNG
COMPOSITIONS ANTI-INSECTES

(30) Priority: 10.05.2006 US 382574
(43) Date of publication of application: 03.09.2008
(73) Proprietor: S.C. Johnson & Son, Inc., Racine, WI 53403 (US)
(72) Inventor: MAGHASI, Anne, T., Racine, WI 53406 (US); NELSON, John, S., Watersmeet, MI 59969 (US); LANCE-GOMEZ, Edward, T., Racine, WI 53402 (US)
(74) Representative: Ruschke, Hans Edvard
(86) International application number: PCT/US2007/011311
(87) International publication number: WO 2007/133638

(56) References cited:
- US-A- 3 445 565
- US-A1- 2005 271 609
- US-B1- 6 441 034
- US-B1- 6 581 807
- US-B1- 6 803 047

## Description

### BACKGROUND OF THE INVENTION

The present invention relates to insect repellent compositions that can be applied to human skin. In particular, the invention relates to sprayable insect repellents that contain a powder for improving skin feel. Furthermore, the invention relates to means for suspending such a powder in a primarily aqueous formulation, optionally together with other liquids or materials not soluble in water, in such a way that the mixture does not readily phase separate, allowing the delivery of all the formulation's ingredients in their proper concentrations without the need for a user's remixing them just prior to delivery.

A variety of insect repellent compositions designed to be applied to the skin have previously been proposed. For example, U.S. Pat. No. 4,774,082 discloses use of N,N-diethyl-m-toluamide ("DEET") as a preferred repellent that can be delivered in a spray form, such as in an aerosol spray, by using hydrocarbon propellants. U.S. Pat. No. 6,719,959 discloses that repellents such as picaridin (or DEET) can be delivered via an aerosol spray or spritz, using materials such as hydrocarbon propellant, water, ethanol, isododecane, and thickeners in the delivery formulation.

It is also known that an insect repellent can be delivered in the form of a gel or otherwise thickened liquid, including where the thickener is a carbomer. See e.g, U.S. Pat. Nos. 6,441,034 and 6,646,011.

As carbomers are typically acidic, a carbomer is preferably used in connection with a neutralizer such as triethanolamine. See also the BF Goodrich web site describing use of AMP-95 as a neutralizer for its Carbopol^{®} brand thickeners.

Powders such as silica powders and corn starch powders have been proposed to be included in aerosol compositions for a variety of reasons. For example, including a powder in such a spray improves skin feel when the composition is sprayed onto the skin. There have even been suggestions to mix such powders with insect repelling compositions. See generally U.S. Pat. No. 6,581,807.

US 3,445,565 discloses a sprayable composition for application to the fur of domestic animals, which consists essentially of a propellent (in a liquefied state under pressure), an active agent, and a carrier in powder form. The powdered carrier helps the active agent to penetrate more deeply.

US 6,803,047 discloses a topical composition containing an aqueous gel which contains a hydrophilic gelling material, said gel having specified rheological properties in terms of viscosity values exhibited under different conditions. The hydrophilic gelling material is a hydrophilic gelling polymer chosen from at least one water-soluble and water-dispersible terephthalic copolyester oligomer comprising dicarboxylate repeating units of a formula shown in the document.

From US 2005/0271609 other compositions containing a gelling agent are known, especially for treating the underarm and thus also containing a deodorant and/or odour reducing agent. The gelling agent can be clay or laponite.

However, powders included in sprayable formulations tend to settle out, thus requiring a user to shake the container before each spray (or risk having formulations with different powder content as the product is used up). Regardless of whether consumers remember to shake the container before use, requiring such shaking often also leads to some consumer dissatisfaction. Complicating matters is that if such powders are incorporated into sprays the overall formulation may lead to clogging of the spray nozzle if poorly dispersed powder is directed through the nozzle.

Accordingly, a need exists for improved, powder-containing, insect repellent formulations that avoid the need for shaking the formulation immediately prior to use, while also avoiding significant clogging problems, optionally together with the stable distribution of other beneficial ingredients not soluble in water..

### BRIEF SUMMARY OF THE INVENTION

The present invention provides insect repellent compositions that provide improved skin feel, such as a dry, non-greasy/non-oily skin feeL There is provided a powder ingredient where the powder is essentially permanently suspended in the composition so that no shaking to resuspend settled out powder is necessary immediately before the composition is applied to the skin.

The invention provides a sprayable insect repellent composition. There is a liquid carrier which comprises water and an organic solvent selected from the group consisting of alcohols an insect repellent, a powder, a carbomer thickener and a neutralizer selected from either organic or inorganic bases. The thickener is present in a sufficient amount so as to suspend the powder in the carrier, yet not so high an amount so as to prevent the composition from being sprayed.

Highly preferred liquid carriers include water and a short chain monohydric alcohol such as ethanol.

The thickeners are gelling thickeners capable of shear thinning when, for example, being delivered via a spray nozzle. The thickeners are capable of forming a thixotropic gel and are carbomer thickeners.

When the thickener is too acidic for the proposed use absent neutralization, it is desirable to control the pH with a neutralizer.

Particularly preferred ingredient ranges are:

(a) 5% by weight to 60% by weight of water;

(b) up to 50 % by weight of alcohol;

(c) 0% by weight to 65% by weight of hydrocarbon;

(d) 1% by weight to 20% by weight of insect repellent;

(e) 0.5% by weight to 25% by weight of powder, and

(f) 0.05% by weight to 1% by weight of carbomer thickener.

When the composition is in a form of an aerosol spray, a propellant gas is also included. Particularly preferred propellant gasses are selected from the group consisting of hydrocarbon gasses, hydrofluorocarbon gasses, and chlorofluorocarbon gasses. For example, the gasses may be mixtures of ethane, propane, butane, isobutane, pentane, isopentane, or a subset thereof.

The composition may also include a fragrance, and/or other skin care ingredients of interest. For example, sunscreen, waterproofing, emollient, or other chemicals of interest for treating the skin may be included where otherwise compatible with the core ingredients.

In another form the invention provides a canister capable of delivering an aerosolized spray of insect repellent. The canister has a housing having an internal cavity containing an insect repellent composition, and a control valve for delivering the repellent from the cavity to a selected site. The composition includes a liquid carrier, an insect repellent, a gas propellant, a powder, and a carbomer thickener. The thickener is present in a sufficient amount so as to suspend the powder in the carrier, yet not so high an amount so as to prevent the composition from being sprayed through the control valve.

In yet another form the invention provides a method for repelling insects from human skin. One sprays such a composition onto the skin.

The inventors have discovered that water acts with their thickeners to form a gel structure to suspend the powder. They have further learned that their other proposed ingredients do not destroy the gel structure.

It will be appreciated that the present invention provides insect repellent compositions with good skin feel and little risk of clogging the spray dispenser. These and still other advantages of the present invention will be apparent from the description below.

### DETAILED DESCRIPTION OF THE PREFERRED EMBODIMENTS

There are provided insect repellent compositions that contain water, an organic solvent selected from the group consisting of alcohols (preferably ethanol, optionally also with isododecane or other skin feel affecting organic solvents), an insect repellent active, preferably DEET or picaridin, a powder selected from the group consisting of starch powders (preferably corn starch), a carbomer thickener, a neutralizer (preferably triethanolamine or 2-amino-2-methyl-1-propanol), and, when the formula is an aerosol spray, a gas propellant (preferably a hydrocarbon gas).

The composition may further contain fragrances, colorants, other suspended solids or materials not completely dissolved in the water and organic solvent, and other compatible materials. The powder ingredient provides an improved skin feel and the thickener ingredient serves the function of essentially permanently suspending the powder in the composition via a gel structure so that no shaking to resuspend settled out powder is necessary immediately before the composition is applied to the skin. Preferably the gel is thixotropic. Surprisingly, the gel structure does not clog standard spritz pump or aerosol spray valves.

For the purpose of the present invention, "insect" means not only flying insects, but also crawling insects. It also includes as well spiders, millipedes, and other arthropods commonly controlled in essentially the same manner or with the same agents as are commonly used to control insects pests.

"Insect repellent" means any chemical that prevents or discourages insects from contacting a treated site (e.g. human skin treated with an affective amount of the repellent), or remaining close to a treated site. The particular insects that are repelled by the composition of the invention will depend upon the insect repellent active material selected. While some insect repellent active materials may be specific to a particular insect species, other active materials may broadly repel a variety of insects.

Among repellents that can be employed are N,N diethyl-m-toluamide ("DEET"), hydroxy-ethyl isobutyl piperidine carboxylate (1-piperidinecarboxylic acid or picaridin, Bayer KBR 3023), ethyl butylacetylaminopropionate (IR3535 by Merck Co.), 2-ethyl-1,3-hexanediol, oil of citronella, soy bean oil, lemon grass oil, geranium/geraniol oil, p-menthane-3,8-diol, and mixtures thereof. Other actives that can be used in the present invention are disclosed in U.S. Pat. Nos. 5,130,136 and 5,698,209. In some preferred embodiments the amount of the one or more insect repellent actives is from 1% to 20%, preferably from 2% to 17%, and more preferably from 2% to 15% by weight based on the total weight of the composition.

Water used in the present invention may be for example purified water, deionized water, or the like. The amount of water included in the composition of the invention may be from 5% to 60%, preferably from 10% to 55%, and more preferably from 10% to 50% by weight based on the total weight of the composition.

Preferred alcohols are represented by the formula R-OH wherein R is a hydrocarbon chain of 1 to 8, preferably 1 to 6 more preferably 2 to 4. Most preferred is ethanol. The hydrocarbon chain can be saturated, unsaturated, linear, branched, cyclic, or polycyclic and can have substituted groups including those with heteroatoms (atoms other than carbon and hydrogen).

Isododecane is another preferred ingredient in the composition of the invention when a quickly evaporating alcohol is used. Isododecane provides an improved skin feel when the composition is, for example, a topical insect repellent. In this context, isododecane is a solvent of reduced volatility, compared to an alcohol of the chain length referred to above. Similar, alternative solvents or organic liquids can be used, when otherwise suitable for topical application. Examples include C₁₀ to C₂₀ saturated hydrocarbons with or without monomethyl branching.

The amount of total organic solvents included in the composition of the invention (apart from propellant gas) is preferably from 15% to 65%, more preferably from 20% to 60%, and most preferably from 25% to 57% by weight based on the total weight of the composition.

The powders used in the composition of the invention may be those for improving skin fell, such as providing a dry fell, when composition is applied to the skin. A powder selected from the group consisting of starch powders traditionally used to deliver a dry feel to the skin is used in the invention. In addition or in alternative, the powder may provide other functions such as holding the repellent active to the skin to maintain its effect or acting as an active.

In one embodiment, one or more starch powders such as corn, barley, tapioca, or wheat powders are used in the present invention. By "starch," we mean a polymer of glucose linked by α(1-4) Linkages to give linear chains, which are joined by α(1-6) linkages resulting in branches in the polymer. Normal starch contains about 75% amylopectin, a highly-branched molecule, and 25% of amylose, a primarily linear molecule. These polymers are organized into an insoluble granule within cereal seeds. Starch granules are highly organized, containing of a series of concentric spheres with alternating crystalline and amorphous regions. These may also be chemically-modified starches.

Corn starch powder is a preferred powder for the purposes of the present invention. The total amount of one or more powders that may be included in the composition of the invention is preferably from 0.5% to 25%, more preferably from 1% to 20%, and most preferably from 1% to 15% by weight based on the total weight of the composition.

The thickener that is sufficiently thick to keep the powder permanently suspended in the composition yet is capable of shearing is a carbomer. "Carbomers" are thickening polymers of acrylic acid, methacrylic acid, or salts thereof, that are cross-linked. This cross-linking can be with a polyfunctional compound such as polyalkenyl ethers of sugars or of polyalcohols (e.g., allyl ether of sucrose, allyl ether of pentaeythritol, or allyl ether of propylene). Carbomers are especially useful in a spray composition of the invention because they thin sufficiently under shear force, e.g., when sprayed, so as not to clog the sprayer during the spray. In one embodiment, homopolymers or copolymers of acrylic acid or salts thereof cross-linked with an allyl ether of pentaeythritol, an allyl ether of sucrose, or an allyl ether of propylene are used in the present invention. An exemplary carbomer useful in the compositions of the invention is a copolymer of C₁₀₋₃₀ alkyl acrylates and one or more monomers of acrylic acid, methacrylic acid or one of their simple esters cross-linked with an allyl ether of sucrose or an allyl ether of pentaerythritol.

Specific examples of carbomers useful in the present invention include the products sold under the name Carbopol^{®} (BF Goodrich, Ohio, USA) such as Carbopol 934, Carbopol 940, Carbopol 941, Carbopol 971, Carbopol 974, Carbopol EZ2, Carbopol ETD 2001, Carbopol ETD 2020, Carbopol ETD 2050, Carbopol ultrez 10, Carbopol ultrez 20, or Carbopol ultrez 21. The total amount of one or more such thickener that may be included in the composition of the invention is preferably from 0.05% to 1%, more preferably from 0.1% to 0.5%, and most preferably from 0.15% to 0.4% by weight based on the total weight of the composition.

If a thickener is too acidic (or too basic) for the desired application (e.g. on human skin) it can be neutralized to a desired pH range around the neutral pH of 7.0. For example, the composition may have a pH in the range of 5.0 to 9.0, preferably from 6.0 to 8.0, and more preferably from 6.5 to 7.5.

Carbomers are acidic and they can be neutralized with either organic or inorganic bases. Exemplary organic and inorganic bases include 2-amino-2-methyl-1-propanol 95% (AMP-95), triethanolamine, tris(hydroxymethyl)amino methane (tromethamine), tetrahydroxypropyl ethylenediamine, PEG-15 cocamine, diisopropanolamine, triisopropanolamine, arginine, ammonium hydroxide, sodium hydroxide, and potassium hydroxide. The total amount of one or more gel thickener neutralizers that may be included in a composition of the invention is preferably from 0.05% to 1%, more preferably from 0.1% to 0.5%, and most preferably from 0.15% to 0.4% by weight based on the total weight of the composition.

The compositions of the invention are designed to be sprayed, such as in an aerosol spray or a pump spritz spray. When they are used as an aerosol spray there will be a gas propellant (e.g., hydrocarbons, hydrofluorocarbons, chlorofluorocarbons, or a mixture thereof) provided in the spray composition. An exemplary insect repellent aerosol spray of the invention contains an amount of water ranging from 10% to 50% by weight of the total weight of the composition, an amount of one or more organic solvents ranging from 25% to 57% by weight of the total weight of the composition, an amount of one or more repellent actives ranging from 1% to 20% by weight of the total weight of the composition, an amount of one or more powders ranging from 1% to 15% by weight of the total weight of the composition, an amount of one or more gel thickeners ranging from 0.1% to 0.5% by weight of the total weight of the composition, an amount of one or more gel thickener neutralizers ranging from 0.1% to 0.5% by weight of the total weight of the composition, and an amount of a propellant gas ranging from 5% to 15% by weight of the total weight of the composition

The spray may also be a pump spritz spray. An exemplary insect repellent pump spritz spray of the invention contains an amount of water ranging from 25% to 55% by weight of the total weight of the composition, an amount of one or more organic solvents ranging from 20% to 60% by weight of the total weight of the composition, an amount of one or more repellent actives ranging from 1% to 20% by weight of the total weight of the composition, an amount of one or more powders ranging from 1% to 20% by weight of the total weight of the composition, an amount of one or more gel thickeners ranging from 0.1% to 0.4% by weight of the total weight of the composition, and optionally an amount of one or more gel thickener neutralizers ranging from 0.1% to 0.4% by weight of the total weight of the composition.

The order of mixing the spray components is not critical, albeit mixing the powder in should be done in a way so as to minimize lump formation. Propellant gas can be added in a conventional manner.

By way of example, but not limitation, examples of the present invention are provided below in the following eight sets of examples:

Example 1- Nine examples of insect repellent aerosol spray compositions (1-9) of the invention are provided as follows:

| | 1 | 2 | 3 | 4 | 5 | 6 | 7 | 8 | 9 |
|---|---|---|---|---|---|---|---|---|---|
| | % wt. | % wt. | % wt | % wt. | %wt. | % wt. | % wt. | % wt. | %wt. |
| water | 25.00 | 45.00 | 35.00 | 35.00 | 45.00 | 25.00 | 25.00 | 35.00 | 45.00 |
| ethanol | 50.62 | 30.62 | 43.12 | 45.62 | 35.62 | 55.62 | 53.12 | 40.62 | 33.12 |
| DEBT | 7.00 | 7.00 | 7.00 | 7.00 | 7.00 | 7.00 | 7.00 | 7.00 | 7.00 |
| corn starch | 7.00 | 7.00 | 4.50 | 2.00 | 2.00 | 2.00 | 4.50 | 7.00 | 4.50 |
| Carbopol ETD 2020 | 0.20 | 0.20 | 0.20 | 0.20 | 0.20 | 0.20 | 0.20 | 0.20 | 0.20 |
| AMP-95 | 0.18 | 0.18 | 0.18 | 0.18 | 0.18 | 0.18 | 0.18 | 0.18 | 0.18 |
| hydrocarbon propellant | 10.00 | 10.00 | 10.00 | 10.00 | 10.00 | 10.00 | 10.00 | 10.00 | 10.00 |

Example 2 - Nine more aerosol examples with the same ingredients except for a different neutralizer:

| | 1 | 2 | 3 | 4 | 5 | 6 | 7 | 8 | 9 |
|---|---|---|---|---|---|---|---|---|---|
| | % wt. | % wt. | % wt. | % wt. | % wt. | % wt. | % wt. | % wt. | % wt. |
| water | 45.00 | 25.00 | 35.00 | 35.00 | 45.00 | 25.00 | 25.00 | 35.00 | 45.00 |
| ethanol | 30.50 | 50.50 | 43.00 | 45.50 | 35.50 | 55.50 | 53.00 | 40.50 | 33.00 |
| DEET | 7.00 | 7.00 | 7.00 | 7.00 | 7.00 | 7.00 | 7.00 | 7.00 | 7.00 |
| corn starch | 7.00 | 7.00 | 4.50 | 2.00 | 2.00 | 2.00 | 4.50 | 7.00 | 4.50 |
| Carbopol ETD 2020 | 0.20 | 0.20 | 0.20 | 0.20 | 0.20 | 0.20 | 0.20 | 0.20 | 0.20 |
| TEA | 0.30 | 0.30 | 0.30 | 0.30 | 0.30 | 0.30 | 0.30 | 0.30 | 0.30 |
| hydrocarbon propellant | 10.00 | 10.00 | 10.00 | 10.00 | 10.00 | 10.00 | 10.00 | 10.00 | 10.00 |

Example 3 - Three more aerosol examples with the same ingredients as Example 1 except for a different active:

| | 1 (% by weight) | 2 (% by weight) | 3 (% by weight) |
|---|---|---|---|
| water | 25.00 | 35.00 | 20.0.0% |
| ethanol | 39.43 | 29.62 | 44.62 |
| p-menthane-3,8-diol | 15.00 | 15.00 | 15.00 |
| corn starch | 10.00 | 10.00 | 10.00 |
| Carbopol ETD 2020 | 0.30 | 0.20 | 0.20 |
| AMP-95 | 0.27 | 0.18 | 0.18 |
| hydrocarbon propellant | 10.00 | 10.00 | 10.00 |

Example 4 - Three more aerosol examples:

| | 1 (% by weight) | 2 (% by weight) | 3 (% by weight) |
|---|---|---|---|
| water | 30.00 | 41.42 | 25.00 |
| ethyl alcohol | 39.42 | 28.00 | 49.42 |
| picardin | 10.33 | 10.33 | 10.33 |
| corn starch | 10.00 | 10.00 | 5.00 |
| Carbopol ultrez 10, Carbopol ultrez 21, or Carbopol ETD 2020 | 0.10 | 0.10 | 0.10 |
| triethanolamine | 0.15 | 0.15 | 0.15 |
| hydrocarbon propellant | 10.00 | 10.00 | 10.00 |

Example 5 - Three pump spritz examples:

| | 1 (% by weight) | 2 (% by weight) | 3 (% by weight) |
|---|---|---|---|
| water | 36.83 | 44.45 | 35.00 |
| ethanol | 37.62 | 30.00 | 49.45 |
| picardin | 5.17 | 5.17 | 5.17 |
| corn starch | 5.00 | 5.00 | 5.00 |
| Carbopol ETD 2020 | 0.20 | 0.20 | 0.20 |
| AMP-95 | 0.18 | 0.18 | 0.18 |
| isododecane | 15.00 | 15.00 | 5.00 |

Example 6 - Two more pump spritz examples, albeit with a different active:

| | 1 (% by weight) | 2 (% by weight) |
|---|---|---|
| water | 50.00 | 35.00 |
| ethanol | 37.62 | 37.43 |
| ethyl butylacetylaminopropionate | 7.00 | 7.00 |
| corn starch | 5.00 | 5.00 |
| Carbopol ETD 2020 | 0.20 | 0.30 |
| AMP-95 | 0.18 | 0.27 |
| isododecane | 0.00 | 15.00 |

Example 7 - Two more pump spritz examples:

| | 1 (% by weight) | 2 (% by weight) |
|---|---|---|
| water | 35.00 | 35.00 |
| ethanol | 50.62 | 22.62 |
| DEET | 2.00 | 15.00 |
| corn starch | 2.00 | 12.00 |
| Carbopol ETD 2020 | 0.20 | 0.20 |
| AMP-95 | 0.18 | 0.18 |
| isododecane | 10.00 | 15.00 |

Example 8 - Two more pump spritz examples:

| | 1 (% by weight) | 2 (% by weight) |
|---|---|---|
| water | 50.00 | 35.00 |
| ethanol | 37.62 | 37.43 |
| p-menthane-3,8-diol | 7.00 | 7.00 |
| corn starch | 5.00 | 5.00 |
| Carbopol ETD 2020 | 0.20 | 0.30 |
| AMP-95 | 0.18 | 0.27 |
| isododecane | 0.00 | 15.00 |

The nature of the dispenser is not critical. One aerosol dispenser that is designed for delivering a powdered spray is disclosed in U.S. Pat. No. 6,581,807, the disclosure of which is hereby incorporated by reference as if fully set forth herein. The base formulation and propellant gas of the present invention would be loaded into those dispensers in a conventional manner.

In the case of a spritz spray the formulation would be poured into a conventional pump spritz bottle. The nature of the pump spritz bottle is not critical, and a wide variety of conventional spritz bottles currently used to deliver DEET or other insecticidal formulations could be used.

In the most preferred manner the sprayer would be held about six inches from human skin and the spray then applied to that skin. This could, for example, provide mosquito repellency.

While preferred embodiments of the present invention have been described and otherwise disclosed herein, alternative embodiments are also intended to be within the scope of the claims. Thus, the invention is not to be judged solely by the preferred embodiments. Rather, the claims should be looked to in order to judge the full scope of the invention.

### Industrial Applicability

The invention provides sprayable insect control compositions which do not require shaking immediately prior to dispensing, notwithstanding the presence of powders in the compositions which provide skin feel benefit.

## Claims

1. A sprayable insect repellent composition, comprising:
a liquid carrier which comprises water and an organic solvent selected from the group consisting of alcohols;
an insect repellent;
a powder selected from the group consisting of starch powders;
a carbomer thickener, wherein the thickener is present in a sufficient amount so as to suspend the powder in the carrier, yet not so high an amount so as to prevent the composition from being sprayed; and
a neutralizer selected from either organic or inorganic bases.

2. The composition of claim 1, wherein the group of starch powders consists of corn, barley, tapioca, and wheat starch powders, preferably corn starch powder.

3. The composition of claim 1 or 2, wherein the neutralizer is selected from the group consisting of 2-amino-2-methyl-1-propanol, triethanolamine, tetrahydroxypropyl ethylenediamine, tromethamine, PEG-15 cocamine, diisopropanolamine, triisopropanolamine, arginine, ammonium hydroxide, sodium hydroxide, and potassium hydroxide.

4. The composition of any of claims 1 to 3, wherein the composition comprises:
(a) 5% by weight to 60% by weight of water;
(b) up to 50% by weight of alcohol;
(c) 0% by weight to 65% by weight of hydrocarbon;
(d) 1% by weight to 20% by weight of insect repellent;
(e) 0.5% by weight to 25% by weight of powder; and
(f) 0.05% by weight to 1% by weight of carbomer thickener.

5. The composition of claim 4, wherein the hydrocarbon is selected from the group consisting of C₁₀ to C₂₀ saturated hydrocarbons with or without monomethyl branching, preferably is isododecane.

6. The composition of any of claims 1 to 5, wherein the organic solvent is selected from the group consisting of monohydric alcohols having from 1 to 8, preferably from 1 to 6, more preferably from 2 to 4 carbon atoms, most preferably is ethanol.

7. The composition of any of claims 4 to 6, wherein the organic solvent is ethanol with isododecane.

8. The composition of any of claims 1 to 7, wherein the insect repellent is selected from the group consisting of N,N-diethyl-m-toluamide, picaridin, p-menthane-3,8-diol, and ethyl butylacetylaminopropionate.

9. The composition of any of claims 1 to 8 further comprising a fragrance.

10. The composition of any of claims 1 to 9, wherein the thickener forms a thixotropic gel.

11. The composition of any of claims 1 to 10, wherein the composition is in a form of an aerosol spray, and the composition further comprises a propellant gas.

12. The composition of claim 11, wherein the propellant gas is selected from the group consisting of hydrocarbon gasses, hydrofluorocarbon gasses, and chlorofluorocarbon gasses.

13. The composition of any of claims 1 to 10, wherein the composition is in a form of a pumpable spritz spray.

14. A canister capable of delivering an aerosolized spray of insect repellent, said canister comprising:
a housing having an internal cavity containing an insect repellent composition which is as defined in claims 11 or 12; and
a control valve for delivering the repellent composition from the cavity to a selected site.

15. A method for repelling insects from human skin comprising the step of spraying onto the skin a composition as defined in any of claims 1 to 13.

## Patentansprüche

1. Insekten abweisendes sprühbares Mittel, das aufweist:
einen flüssigen Träger, der Wasser sowie ein organisches Lösungsmittel aus der Gruppe der Alkohole enthält;
ein Insekten-Abwehrmittel;
ein Pulver aus der Gruppe der Stärkepulver;
ein Carbomer-Verdickungsmittel in einer Menge, die ausreicht, um das Pulver im Träger zu suspendieren, aber nicht so groß ist, dass sie ein Versprühen des Mittels verhindert; und
einen aus organischen oder anorganischen Basen gewählten Neutralisator.

2. Mittel nach Anspruch 1, bei dem die Gruppe der Stärkepulver aus Mais-, Gerste-, Tapioca- und Weizen-Stärkepulvern besteht, wobei es sich vorzugsweise um Maisstärke-Pulver handelt.

3. Mittel nach Anspruch 1 oder 2, bei dem der Neutralisator gewählt ist aus der Gruppe bestehend aus 2-Amino-2-methyl-1-propanol, Triethanolamin, Tetrahydroxypropylethylendiamin, Trimethamin, PEG-15-Cocamin, Diisopropanolamin, Triisopropanolamin, Arginin, Ammoniumhydroxid, Natriumhydroxid und Kaliumhydroxid.

4. Mittel nach einem der Ansprüche 1 bis 3, das aufweist:
(a) 5 Gew.-% bis 60 Gew.-% Wasser;
(b) bis 50 Gew.-% Alcohol;
(c) 0 Gew.% bis 65 Gew.-% Kohlenwasserstoff;
(d) 1 Gew.-% bis 20 Gew.-% Insekten-Abwehrmittel;
(e) 0,5 Gew.-% bis 25 Gew.-% Pulver; und
(f) 0,05 Gew.-% bis 1 Gew.-% Carbomer-Verdickungsmittel.

5. Mittel nach Anspruch 4, bei dem der Kohlenwasserstoff aus der Gruppe der gesättigten C₁₀- bis C₂₀-Kohlenwasserstoffen mit oder ohne Monomethyl-Verzweigung besteht und vorzugsweise Isododecan ist.

6. Mittel nach einem der Ansprüche 1 bis 5, bei dem das organische Lösungsmittel aus der Gruppe der monohydrischen Alcohole mit 1 bis 8, besser 1 bis 6 und vorzugsweise 2 bis 4 C-Atomen gewählt und am besten Ethanol ist.

7. Mittel nach einem der Ansprüche 4 bis 6, bei dem das organische Lösungsmittel Ethanol mit Isododecan ist.

8. Mittel nach einem der Ansprüche, bei dem das Insekten-Abwehrmittel aus der Gruppe N,N-Diethyl-m-toluamid, Picaridin, p-Menthan-3,8-diol und Ethylbutylacetylaminopropionat gewählt ist.

9. Mittel nach einem der Ansprüche 1 bis 8, das weiterhin einen Duftstoff aufweist.

10. Mittel nach einem der Ansprüche 1 bis 9, bei dem das Verdickungsmittel ein thixotropes Gel bildet.

11. Mittel nach einem der Ansprüche 1 bis 10, das in Form eines Aerosol-Sprays vorliegt, und das Mittel weiterhin ein Treibgas aufweist.

12. Mittel nach Anspruch 11, bei dem das Treibgas aus der Gruppe der Kohlenwasserstoff-, Hydrofluorkohlenwasserstoff- und Fluorchlorkohlenwasserstoff-Gase gewählt ist.

13. Mittel nach einem der Ansprüche 1 bis 10, das in Form eines pumpbaren Spritz-Sprays vorliegt.

14. Kanister, mit dem sich ein aerosolisiertes Insekten-Abwehrmittel ausgeben lässt, und der aufweist:
ein Gehäuse mit einem Innenraum, der ein Insekten-Abwehrmittel nach Anspruch 11 oder 12 enthält; und
ein Steuerventil zur Ausgabe des Abwehrmittels aus dem Innenraum an einen gewählten Ort.

15. Verfahren zum Abweisen von Insekten von der menschlichen Haut durch Aufsprühen eines Mittels nach einem der Ansprüche 1 bis 13 auf diese.

## Revendications

1. Composition de répulsif pour insectes pulvérisable, comprenant :
un véhicule liquide qui comprend de l'eau et un solvant organique sélectionné dans le groupe constitué des alcools ;
un répulsif pour insectes ;
une poudre sélectionnée dans le groupe constitué des poudres d'amidon ;
un épaississant carbomère, où l'épaississant est présent en une quantité suffisante pour mettre la poudre en suspension dans le véhicule mais pas en une quantité si élevée qu'elle empêche la pulvérisation de la composition ; et
un neutralisant sélectionné parmi des bases soit organiques, soit inorganiques.

2. Composition selon la revendication 1, où le groupe de poudres d'amidon est constitué de poudres d'amidon de maïs, d'avoine, de tapioca et de froment, de préférence de poudre d'amidon de maïs.

3. Composition selon la revendication 1 ou 2, où le neutralisant est sélectionné dans le groupe constitué du 2-amino-2-méthyl-1-propanol, de la triéthanolamine, de la tétrahydroxypropyléthylènediamine, de la trométhanine, de la PEG-15 cocamine, de la diisopropanolamine, de la triisopropanolamine, de l'arginine, de l'hydroxyde d'ammonium, de l'hydroxyde de sodium et de l'hydroxyde de potassium.

4. Composition selon l'une quelconque des revendications 1 à 3, où la composition comprend :
(a) 5 % en poids à 60 % en poids d'eau ;
(b) jusqu'à 50 % en poids d'alcool ;
(c) 0 % en poids à 65 % en poids d'hydrocarbure ;
(d) 1 % en poids à 20 % en poids de répulsif pour insectes ;
(e) 0,5 % en poids à 25 % en poids de poudre ; et (f) 0,05 % en poids à 1 % en poids d'épaississant carbomère.

5. Composition selon la revendication 4, où l'hydrocarbure est sélectionné dans le groupe constitué des hydrocarbures saturés en C₁₀ à C₂₀, avec ou sans ramification monoéthyle, et est de préférence de l'isododécane.

6. Composition selon l'une quelconque des revendications 1 à 5, où le solvant organique est sélectionné dans le groupe constitué des alcools monovalents ayant de 1 à 8, de préférence de 1 à 6, de façon préférée de 2 à 4 atomes de carbone, et est de façon particulièrement préférée de l'éthanol.

7. Composition selon l'une quelconque des revendications 4 à 6, où le solvant organique est de l'éthanol avec de l'isododécane.

8. Composition selon l'une quelconque des revendications 1 à 7, où le répulsif pour insectes est sélectionné dans le groupe constitué du N,N-diéthyl-m-toluamide, de la picaridine, du p-menthane-3,8-diol et du butylacétylaminopropionate d'éthyle.

9. Composition selon l'une quelconque des revendications 1 à 8, comprenant en outre un parfum.

10. Composition selon l'une quelconque des revendications 1 à 9, où l'épaississant forme un gel thixotrope.

11. Composition selon l'une quelconque des revendications 1 à 10, où la composition se présente sous la forme d'un spray aérosol et la composition comprend en outre un gaz propulseur.

12. Composition selon la revendication 11, où le gaz propulseur est sélectionné dans le groupe constitué des gaz hydrocarbures, des gaz hydrofluorocarbures et des gaz chlorofluorocarbures.

13. Composition selon l'une quelconque des revendications 1 à 10, où la composition se présente sous la forme d'un spray de pulvérisation pompable.

14. Bidon capable de dispenser un spray aérosol de répulsif pour insectes, ledit bidon comprenant :
un boîtier ayant une cavité interne contenant une composition de répulsif pour insectes selon l'une quelconque des revendications 11 et 12 ; et
une soupape de commande pour dispenser la composition de répulsif de la cavité à un site sélectionné.

15. Procédé de répulsion des insectes de la peau humaine, comprenant l'étape de pulvérisation sur la peau d'une composition selon l'une quelconque des revendications 1 à 13.
